(19) Europäisches Patentamt
European Patent Office
Office européen des brevets

(11) Publication number: **0 202 062**
**A2**

# (12) EUROPEAN PATENT APPLICATION

(21) Application number: 86303404.7

(22) Date of filing: 06.05.86

(51) Int. Cl.⁴: C 07 D 453/02
A 61 K 31/435

(30) Priority: 11.05.85 GB 8511988

(43) Date of publication of application:
20.11.86 Bulletin 86/47

(84) Designated Contracting States:
BE CH DE FR GB IT LI NL

(71) Applicant: BEECHAM GROUP PLC
Beecham House Great West Road
Brentford Middlesex TW8 9BD(GB)

(72) Inventor: King, Francis David
8 Heath Row Bishop's Stortford
Hertfordshire CM23 5EF(GB)

(74) Representative: Jones, Pauline et al,
Beecham Pharmaceuticals Patents & Trade Marks Dept.
Great Burgh Yew Tree Bottom Road
Epsom Surrey KT18 5XQ(GB)

(54) **Quinuclidine derivatives.**

(57) Compounds of formula (I), and pharmaceutically acceptable salts thereof:

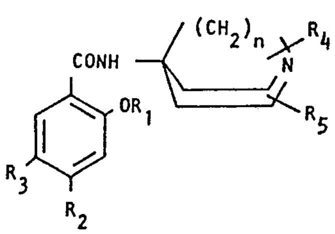

(I)

wherein

$R_1$ is $C_{1-6}$ alkyl;

$R_2$ is amino, $C_{1-7}$ acylamino or methyl;

$R_3$ is halo or $C_{1-6}$ alkylthio;

$R_4$ and $R_5$ are independently selected from hydrogen, $C_{1-6}$ alkyl, phenyl or phenyl $C_{1-4}$ alkyl any of which phenyl moieties may be substituted by one or two of halo, $CF_3$, $C_{1-6}$ alkoxy or $C_{1-6}$ alkyl; and

n is 1 to 3

having gastric motility enchancing activity, a process for their preparation and their use as pharmaceuticals.

EP 0 202 062 A2

TITLE MODIFIED
see front page

0202062

B1811

# NOVEL COMPOUNDS

This invention relates to substituted benzamides having pharmacological activity, to a process for their preparation and to their use as pharmaceuticals.

U.K. Patent Application GB 2125398A discloses benzamides and benzoates having a quinuclidinyl side chain, especially 3-quinuclidinyl, and having serotonin M-antagonist activity. EP-A-99789 discloses N-3-quinuclidinyl benzamides having gastro-intestinal motility enhancing activity.

A group of compounds have now been discovered which are 4-quinuclidinyl and related benzamides which compounds have good gastro-intestinal motility enhancing activity and also anti-emetic activity.

Accordingly, the present invention provides a compound of formula (I) or a pharmaceutically acceptable salt thereof:

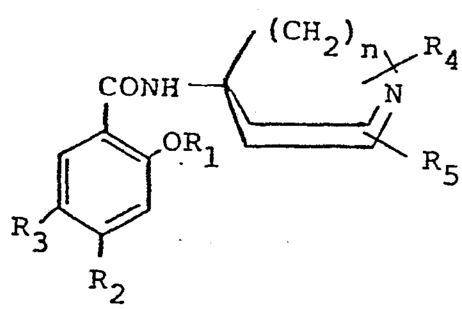

(I)

wherein:

$R_1$ is $C_{1-6}$ alkyl;

$R_2$ is amino, $C_{1-7}$ acylamino or methyl;

$R_3$ is halo or $C_{1-6}$ alkylthio;

$R_4$ and $R_5$ are independently selected from hydrogen, $C_{1-6}$ alkyl, phenyl or phenyl $C_{1-4}$ alkyl any of which phenyl moieties may be substituted by one or two of halo, $CF_3$, $C_{1-6}$ alkoxy or $C_{1-6}$ alkyl; and

n is 1 to 3.

Examples of $R_1$ include methyl, ethyl, n- and iso-propyl. Preferably $R_1$ is methyl.

Examples of $R_2$ include amino and $C_{1-6}$ alkanoylamino, such as formylamino, acetylamino, propionylamino, n- and iso-butyrylamino; and methyl. Often $R_2$ is amino or acetylamino, preferably amino.

Examples of $R_3$ include chloro, bromo, fluoro, methylthio, ethylthio, n- and iso-propylthio. Preferably $R_3$ is chloro or bromo, most preferably chloro.

Examples of $R_4$ and $R_5$ include hydrogen, methyl, ethyl, n- and iso-propyl, n-, iso-, sec- and tert-butyl, and phenyl, benzyl or phenethyl wherein any phenyl moiety is optionally substituted by one or two of chloro, bromo, fluoro, $CF_3$, methoxy, ethoxy, n- or iso-propoxy, methyl, ethyl, n- and iso-propyl. Preferably $R_4$ is hydrogen or methyl, often hydrogen, and $R_5$ is hydrogen.

n may be 1, 2 or 3, preferably 2.

The pharmaceutically acceptable salts of the compounds of the formula (I) include acid addition salts with conventional acids such as hydrochloric, hydrobromic, boric, phosphoric, sulphuric acids and pharmaceutically acceptable organic acids such as acetic, tartaric, maleic, citric, succinic, benzoic, ascorbic, methanesulphonic, α-keto glutaric, α-glycerophosphoric, and glucose-1-phosphoric acids.

The pharmaceutically acceptable salts of the compounds of the formula (I) are usually acid addition salts with acids such as hydrochloric, hydrobromic, phosphoric, sulphuric, citric, tartaric, lactic and acetic acid.

Preferably the acid addition salt is the hydrochloride salt.

Examples of pharmaceutically acceptable salts also include quaternary derivatives of the compounds of formula (I) include the compounds quaternised by compounds such as $R_{10}$-T wherein $R_{10}$ is $C_{1-6}$ alkyl, phenyl-$C_{1-6}$ alkyl or $C_{5-7}$ cycloalkyl, and T is a radical corresponding to an anion of an acid. Suitable examples of $R_{10}$ include methyl, ethyl and *n*- and *iso*-propyl; and benzyl and phenethyl. Suitable examples of T include halide such as chloride, bromide and iodide.

Pharmaceutically acceptable salts of the compounds of formula (I) also include pharmaceutically acceptable N-oxides.

The compounds of the formula (I), and their pharmaceutically acceptable salts, (including quaternary derivatives and N-oxides) may also form pharmaceutically acceptable solvates, such as hydrates, which are included wherever compounds of formula (I) and salts thereof are herein referred to.

It will of course be realised that some of the compounds of the formula (I) have chiral or prochiral centres and thus are capable of existing in a number of stereoisomeric forms including enantiomers. The invention extends to each of these stereoisomeric forms (including enantiomers), and to mixtures thereof (including racemates). The different stereoisomeric forms may be separated one from the other by the usual methods.

A group of compounds within formula (I) is of formula (II):

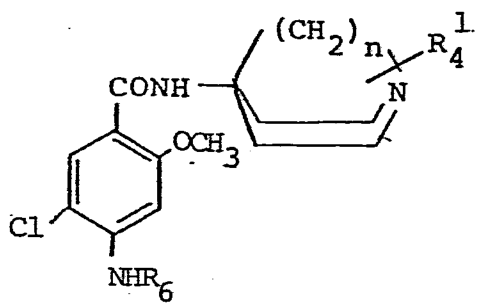

(II)

wherein $R_6$ is hydrogen or acetyl;
$R_4^1$ is hydrogen or $C_{1-6}$ alkyl; and n is as defined in formula (I).

Examples and preferred values of the variable groups are as described for the corresponding groups under formula (I).

The invention also provides a process for the preparation of a compound of formula (I), or a pharmaceutically acceptable salt thereof which process comprises reacting a compound of formula (III):

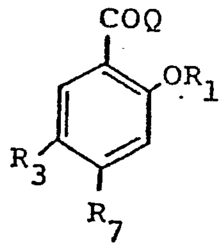

(III)

with a compound of formula (IV):

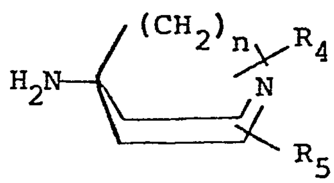

(IV)

wherein Q is a leaving group; $R_7$ is $R_2$ or nitro; and the remaining variables are as hereinbefore defined, and thereafter optionally converting $R_7$ to $R_2$ and/or forming a pharmaceutically acceptable salt.

Examples of leaving groups Q, displaceable by a nucleophile include halogen such as chloro and bromo, hydroxy, carboxylic acyloxy such as $C_{1-4}$ alkanoyloxy or $C_{1-4}$ alkoxycarbonyloxy and activated hydrocarbyloxy such as pentachlorophenoxy.

If a group Q is a halide, then the reaction is preferably carried out at non-extreme temperatures in an inert non-hydroxylic solvent, such as benzene, dichloromethane, toluene, diethyl ether, tetrahydrofuran (THF) or dimethylformamide (DMF). It is also preferably carried out in the presence of an acid acceptor, such as an organic base, in particular a tertiary amine, such as triethylamine, trimethylamine, pyridine or picoline, some of which can also function

as the solvent. Alternatively, the acid acceptor can be inorganic, such as calcium carbonate, sodium carbonate or potassium carbonate. Temperatures of $0^{o}$-$100^{o}C$, in particular 10-$80^{o}C$ are suitable.

If a group Q is hydroxy, then the reaction is generally carried out in an inert non-hydroxylic solvent, such as dichloromethane, THF or DMF optionally in the presence of a dehydrating catalyst, such as a carbodiimide, for example dicyclohexylcarbodiimide. When Y is CO the compound of formula (IV) is preferably in the form of an acid addition salt, such as the hydrohalide, for example the hydrochloride. The reaction may be carried out at any non-extreme temperature, such as -10 to $100^{o}C$, for example, 0 to $80^{o}C$. Generally, higher reaction temperatures are employed with less active compounds whereas lower temperatures are employed with the more active compounds.

If a group Q is carboxylic acyloxy, then the reaction is preferably carried in substantially the same manner as the reaction when $Q_1$ is halide. Suitable examples of acyloxy leaving groups include $C_{1-4}$ alkanoyloxy and $C_{1-4}$ alkoxycarbonyloxy, in which case the reaction is preferably carried out in an inert solvent, such as methylene chloride, at a non-extreme temperature for example ambient temperatures in the presence of an acid acceptor, such as triethylamine. $C_{1-4}$ alkoxycarbonyloxy leaving groups may be generated in situ by treatment of the corresponding compound wherein $Q_1$ is hydroxy with a $C_{1-4}$ alkyl chloroformate.

If a group Q is activated hydrocarbyloxy then the reaction is preferably carried out in an inert polar solvent, such as dimethylformamide. It is also

preferred that the activated hydrocarbyloxy group is a pentachlorophenyl ester and that the reaction is carried out at ambient temperature.

Pharmaceutically acceptable salts of the compounds of this invention may be formed conventionally.

The salts may be formed for example by reaction of the base compound of formula (I) with a pharmaceutically acceptable organic or inorganic acid.

An $R_7$ group may be converted to an amino group by reduction; an $R_7$ $C_{1-7}$ acylamino substituent is convertible to an amino substituent by deacylation, and an amino substituent is convertible to a $C_{1-7}$ acylamino substituent by acylation with a carboxylic acid derivative. The reduction is carried out with a reagent suitable for reducing nitroanisole to aminoanisole; deacylation is carried out by treatment with a base such as an alkali metal hydroxide; acylation is carried out using, for example the corresponding acid chloride or free acid if formylation is to be carried out.

Compounds of the formulae (III) and (IV) are known or are preparable analogously to, or routinely from, known compounds. Compounds of formula (IV) may be prepared in the manner described by H.P. Fischer and C.A. Grob, Helv. Chim. Acta 51, 153, 1968.

Compounds of the present invention have gastro-intestinal motility enhancing activity and therefore may be used in disorders such as retarded gastric emptying, dyspepsia, flatulence, oesophagal reflux and peptic ulcer. The compounds of the present invention also have anti-emetic activity.

The invention also provides a pharmaceutical composition comprising a compound of formula (I), or a pharmaceutically acceptable salt thereof, and a pharmaceutically acceptable carrier.

Such compositions are prepared by admixture and are suitably adapted for oral or parenteral administration, and as such may be in the form of tablets, capsules, oral liquid preparations, powders, granules, lozenges, reconstitutable powders, injectable and infusable solutions or suspensions or suppositories. Orally administrable compositions are preferred, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art, for example with an enteric coating.

Suitable fillers for use include cellulose, mannitol, lactose and other similar agents. Suitable disintegrants include starch, polyvinylpolypyrrolidone and starch derivatives such as sodium starch glycollate. Suitable lubricants include, for example, magnesium stearate.

Suitable pharmaceutically acceptable wetting agents include sodium lauryl sulphate. Oral liquid preparations may be in the form of, for example, aqueous or oily suspensions, solutions, emulsions,

syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

Oral liquid preparations are usually in the form of aqueous or oily suspensions, solutions, emulsions, syrups, or elixirs or are presented as a dry product for reconstitution with water or other suitable vehicle before use. Such liquid preparations may contain conventional additives such as suspending agents, emulsifying agents, non-aqueous vehicles (which may include edible oils), preservatives, and flavouring or colouring agents.

The oral compositions may be prepared by conventional methods of blending, filling or tabletting. Repeated blending operations may be used to distribute the active agent throughout those compositions employing large quantities of fillers. Such operations are, of course, conventional in the art.

For parenteral administration, fluid unit dose forms are prepared containing a compound of the present invention and a sterile vehicle. The compound, depending on the vehicle and the concentration, can be either suspended or dissolved. Parenteral solutions are normally prepared by dissolving the compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the compound is suspended in the vehicle instead of being dissolved and sterilised by exposure of ethylene oxide before suspending in the sterile vehicle. Advantageously, a surfactant or wetting agent is included in the composition to facilitate uniform distribution of the compound of the invention.

The invention further provides a method of treatment or prophylaxis of disorders relating to impaired gastro-intestinal motility and/or emesis in mammals, such as humans, which comprises the administration of an effective amount of a compound of the formula (I) or a pharmaceutically acceptable salt thereof.

An amount effective to treat the disorders hereinbefore described depends on the relative efficacies of the compounds of the invention, the nature and severity of the disorder being treated and the weight of the

mammal. However, a unit dose for a 70kg adult will normally contain 0.5 to 1000mg for example 1 to 500mg, of the compound of the invention. Unit doses may be administered once or more than once a day, for example, 2, 3 or 4 times a day, more usually 1 to 3 times a day, that is in the range of approximately 0.001 to 50 mg/kg/day, more usually 0.002 to 25 mg/kg/day.

No adverse toxicological effects are indicated at any of the aforementioned dosage ranges.

The invention also provides a compound of formula (I) or a pharmaceutically acceptable salt thereof for use as an active therapeutic substance, in particular for use in the treatment of disorders relating to impaired gastro-intestinal motility and/or emesis.

The following Example illustrates the preparation of compounds of formula (I); the following description relates to the preparation of an intermediate.

Description 1

4-Aminoquinuclidine (D.1)

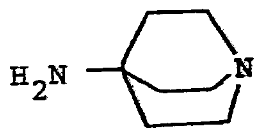

(D1)

A suspension of quinuclidine-4-carboxylic acid (3.0g) in thionyl chloride (20ml) was heated on a steam bath for 2h. The excess thionyl chloride was removed by rotary evaporation and the residue re-evaporated with xylene (2 x 50ml). The residue was stirred with an ice-cold solution of sodium azide (5.0g) in water (50ml) for 10 min, basified with solid $K_2CO_3$ and the acyl azide extracted into ether (3 x 50ml) and dried ($Na_2SO_4$). The filtered solution was carefully evaporated to dryness *in vacuo* and the residue heated to reflux in dry toluene (100ml) for 30 min. The so-formed isocyanate was extracted into 5NHCl (50ml) and heated to reflux for 1h. The aqueous reaction mixture was concentrated *in vacuo* and the residue treated with saturated $K_2CO_3$ solution. The product was extracted into $CH_2Cl_2$ containing 5% ethanol (3 x 50ml), dried ($K_2CO_3$) and evaporation gave the crude 4-aminoquinuclidine (1.5g) which was used without further purification.

Example 1

4-Amino-5-chloro-2-methoxy-N-(4-quinuclidinyl)benzamide (E1)

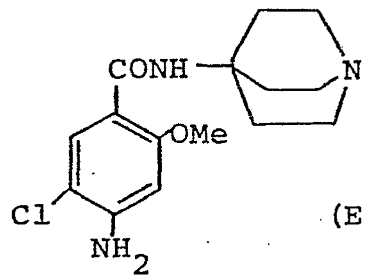

(E1)

A suspension of 4-acetamido-5-chloro-2-methoxybenzoic acid (2.9g) in dry $CH_2Cl_2$ (100ml) was treated with oxalyl chloride (1.0ml) and DMF (5 drops) and stirred at room temperature for 1h. A solution of 4-aminoquinuclidine (1.5g) in $CH_2Cl_2$ (50ml) followed by triethylamine (5ml) was then added and the whole stirred at room temperature for 2h. The $CH_2Cl_2$ solution was washed with 10% $Na_2CO_3$ solution (100ml), dried ($K_2CO_3$), evaporated to dryness and the residue triturated with $Et_2O$ to afford the acetamido derivative of E1 (3.7g). A solution of the acetamido derivative (3.7g) in EtOH (100ml) was treated with 10% NaOH solution (10ml) and heated under reflux for 1h. On cooling, the solvent was removed by rotary evaporation and the residue extracted with $CHCl_3$ (200ml). The $CHCl_3$ extracts were dried ($K_2CO_3$), evaporated to dryness and the residue recrystallised ($CH_2Cl_2$/EtOAc/petrol) to give E1 (1.7g) mp 275-80$^o$ (dec).

$^1H$ NMR (79.5MHz, $d_6$-DMSO/$CDCl_3$)

δ 7.88 (1H, s)
7.51 (1H, brs)
6.50 (1H, s)
5.30 (2H, brs)
3.90 (3H, s)
2.95 (6H, dd)
1.94 (6H, dd)

## Pharmacological Data

### Intragastric pressure in the rat

Intragastric pressure changes were recorded from fasted conscious and restrained rats using a saline filled catheter inserted into the lumen of the stomach via a permanent gastric fistula. The catheter was connected to a physiological pressure transducer and pressure changes recorded on a hot wire pen recorder. An index of activity was obtained by measuring the average height of pressure waves during 10 minute periods. Values for 4 such periods were obtained during assessment of spontaneous activity prior to dosing and for the 40 minute period following dosing with compound or vehicle. The Student's ''t'' test was applied to the mean values obtained for activity prior to and post treatment. Groups of 10 animals were used for each treatment.

The compound of Example 1 was active at a dose of 0.5 mg/kg s.c.

Claims

1. A compound of formula (I), or a pharmaceutically acceptable salt thereof:

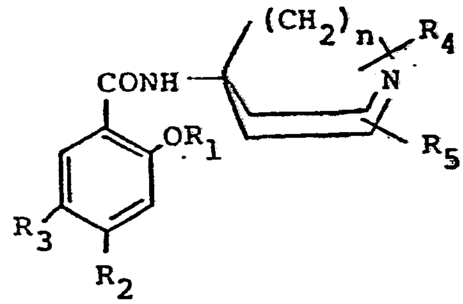

(I)

wherein:

$R_1$ is $C_{1-6}$ alkyl;

$R_2$ is amino, $C_{1-7}$ acylamino or methyl;

$R_3$ is halo or $C_{1-6}$ alkylthio;

$R_4$ and $R_5$ are independently selected from hydrogen, $C_{1-6}$ alkyl, phenyl or phenyl $C_{1-4}$ alkyl any of which phenyl moieties may be substituted by one or two of halo, $CF_3$, $C_{1-6}$ alkoxy or $C_{1-6}$ alkyl; and

n is 1 to 3.

2. A compound according to claim 1 wherein $R_1$ is methyl.

3. A compound according to claim 1 or 2 wherein $R_3$ is chloro or bromo.

4. A compound according to claim 1, 2 or 3 wherein $R_5$ is hydrogen.

5. A compound according to claim 1 of formula (II):

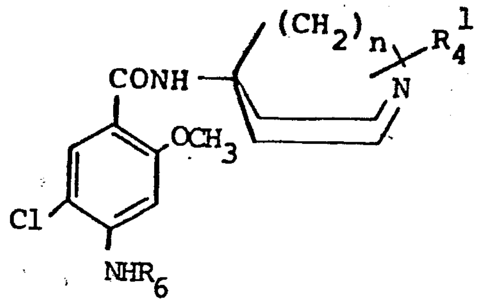

(II)

wherein $R_6$ is hydrogen or acetyl;

$R_4^1$ is hydrogen or $C_{1-6}$ alkyl; and n is as defined in claim 1.

6. A compound according to any one of claims 1 to 5 wherein n is 2.

7. 4-Amino-5-chloro-2-methoxy-N-(4-quinuclidinyl)-benzamide.

8. A process for the preparation of a compound of formula (I) as defined in claim 1, which process comprises reacting a compound of formula (III):

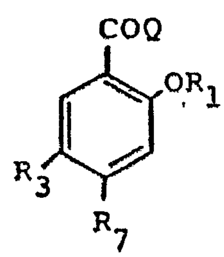

(III)

with a compound of formula (IV):

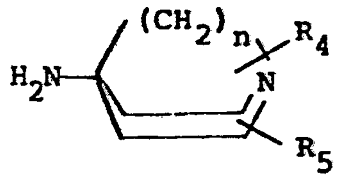

(IV)

wherein Q is a leaving group; $R_7$ is $R_2$ or nitro; and the remaining variables are as defined in claim 1, and thereafter optionally converting $R_7$ to $R_2$ and/or forming a pharmaceutically acceptable salt.

9. A pharmaceutical composition comprising a compound according to any one of claims 1 to 7 or a pharmaceutically acceptable salt therof, and a pharmaceutically acceptable carrier.

10. A compound according to any one of claims 1 to 7 for use in the treatment of disorders relating to impaired gastro-intestinal motility and/or emesis.